# EUROPÄISCHE PATENTSCHRIFT

(11) **EP 0 491 330 B1**
(45) Veröffentlichungstag und Bekanntmachung des Hinweises auf die Patenterteilung: **22.03.1995**
(21) Anmeldenummer: 91121549.9
(22) Anmeldetag: 16.12.1991
(51) Int. Cl.: C07C 61/04, C07C 51/00

(54) **Verfahren zur Herstellung von Dimethylcyclopropancarbonsäure**
Process for the preparation of dimethylcyclopropancarboxylic acid
Procédé pour la préparation de l'acide diméthylcyclopropanecarboxylique

(30) Priorität: 17.12.1990 CH 3991/90
(43) Veröffentlichungstag der Anmeldung: 24.06.1992
(73) Patentinhaber: LONZA AG, CH-4002 Basel (CH)
(72) Erfinder: Meul, Thomas, Dr., Visp (Kanton Wallis) (CH); Kämpfen, Ulrich, Dr., Brig (Kanton Wallis) (CH)
(74) Vertreter: Weinhold, Peter, Dr.

(56) Entgegenhaltungen:
- PATENT ABSTRACTS OF JAPAN, unexamined applications, E Field, Band 7, Nr. 285, 20. Dezember 1983, THE PATENT OFFICE JAPANESE GOVERNMENT, Seite 164 C 201
- CHEMICAL ABSTRACTS, Band 68, Nr. 5, 29. Januar 1968, Columbus, Ohio, USA, Seite 2058, Spalte 1, Zusammenfassung Nr. 21 563e; S. R. LANDOR et al: "Cyclopropanes from alpha,beta-unsaturated esters by the dimethylsulfoxonium methylide reaction"

## Beschreibung

Die Erfindung betrifft ein neues Verfahren zur Herstellung von R,S-2,2-Dimethylcyclopropancarbonsäure, die im folgenden als R,S-2,2-DMCPCS abgekürzt wird.
R,S-2,2-DMCPCS ist ein wichtiges Zwischenprodukt zur Herstellung von S-(+)-2,2-Dimethylcyclopropancarboxamid (abgekürzt im folgenden als S-(+)-2,2-DMCPCA), R,S-2,2,DMCPCS wird durch Racematspaltung in das optisch reine S-(+)-Enantiomere überführt und nachfolgend via Säurechlorid in das S-(+)-2,2-DMCPCA umgewandelt wird (Lit.: EP 093 511). S-(+)-2,2-DMCPCA wiederum dient als Ausgangsmaterial zur Herstellung des Dehydropeptidase-Inhibitors Cilastatin, der in der Therapie zusammen mit Penem- bzw. Carbapenem-Antibiotika verabreicht wird, um die Desaktivierung der Antibiotika durch eine renale Dehydropeptidase in der Niere zu verhindern
(Lit.: EP 048 301).

Aus der Literatur sind mehrere Verfahren zur Herstellung von R,S-2,2-DMCPCS bekannt.

N. Kishner, J.Russ.Phys.Chem.Soc., 45, S.987, 1913, beschreibt eine dreistufige Synthese zur Herstellung von R,S-2,2-DMCPCS ausgehend von Phoron.
In diesem Verfahren wird das Phoron in der ersten Stufe mit Hydrazin in ein Pyrazolinderivat überführt, dieses in der zweiten Stufe mit Kaliumhydroxid, in Gegenwart von Platin, in ein 2,2-Dimethylcyclopropanderivat umgewandelt, welches dann in der dritten Stufe mit Kaliumpermanganat zu R,S-2,2-DMCPCS oxidiert wird.

Nachteil dieses Verfahrens ist, dass die Herstellung des intermediären Pyrazolins mit dem hochgiftigen und kanzerogenen Hydrazin durchgeführt werden muss.

E.R. Nelson et al., J.Am.Chem.Soc., 79, S.3467, 1957, beschreiben ebenfalls ein dreistufiges Verfahren zur Herstellung von R,S-2,2-DMCPCS ausgehend von 2,2-Dimethylpropan-1,3-diol. Dabei wird zunächst das 2,2-Dimethylpropan-1,3-diol mit p-Toluolsulfonylchlorid in das Ditosylatderivat überführt, dieses wird dann in der zweiten Stufe mit Kaliumcyanid in das 2,2-Dimethylcyclopropannitril umgesetzt, welches dann in der dritten Stufe zur entsprechenden Säure hydrolysiert wird.

Ein grosser Nachteil dieses Verfahrens besteht darin, dass dabei grosse Mengen an Kaliumtosylat als Abfallprodukt zu Entsorgung anfallen und mässige Ausbeuten (28%) erreicht werden.

S.R. Landor et al., J.Chem.Soc. (C), S.2495, 1967, beschreiben ein Verfahren zur Herstellung von R,S-2,2-DMCPCS ausgehend von 2-Methylbutensäureethylester, der zunächst mit einem Schwefel-Ylid-Derivat in den 2,2-Dimethylcyclopropancarbonsäureester überführt und dann zur entsprechenden Säure hydrolysiert wird.

Ein grosser Nachteil dieses Verfahren liegt darin, dass der 2,2-Dimethylcyclopropancarbonsäureester in sehr schlechter Ausbeute (9%) erhalten wird und damit die Gesamtausbeute an der entsprechenden Säure noch geringer ist. Ein weiterer Nachteil dieses Verfahrens besteht in der Anwendung des teuren Schwefel-Ylid-Derivats.

Ein weiteres Verfahren zur Herstellung von R,S-2,2-DMCPCS wird in der DE-PS 27 51 133 beschrieben.
In diesem Verfahren wird zunächst der 4-Chlor-4,4-dimethylbuttersäureester aus einem Lactonderivat hergestellt und dieser in Gegenwart von Alkoholaten zum R,S-2,2-Dimethylcyclopropansäureester cyclisiert, der nachfolgend hydrolysiert wird.

Ein grosser Nachteil dieses Verfahrens liegt darin, dass das Lacton kommerziell nicht verfügbar ist und in einer mehrstufigen Synthese hergestellt werden muss.

Aufgabe der Erfindung war es, diese Nachteile auszuschalten und ein einfaches und wirtschaftliches Verfahren zur Herstellung von R,S-2,2-Dimethylcyclopropancarbonsäure zur Verfügung zu stellen, wobei die R,S-2,2-DMCPCS mit guten Ausbeuten erhalten wird.

Diese Aufgabe wurde gemäss Patentanspruch 1 gelöst.

Erfindungsgemäss wird das Verfahren derart durchgeführt, dass man Isobutylenoxid der Formel 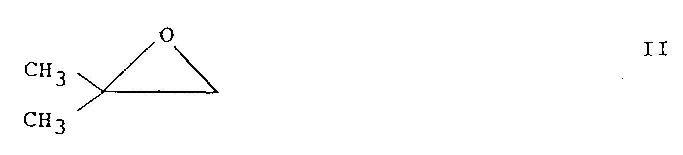
mit einem Phosphonoessigsäuretrialkylester der Formel 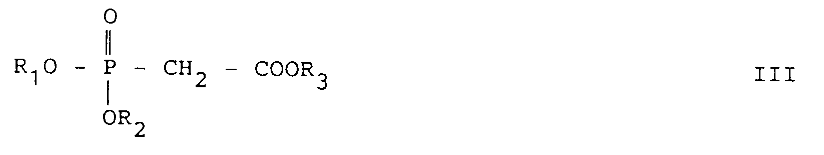
worin R₁, R₂ und R₃ gleich oder verschieden sind und eine C₁-C₄-Alkylgruppe, verzweigt oder unverzweigt, bedeuten, in Gegenwart einer Base in einen R,S-2,2-Dimethylcyclopropancarbonsäure-C₁-C₄-alkylester der allgemeinen Formel 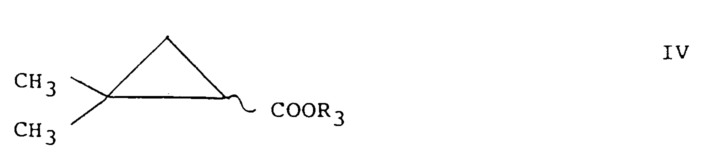
worin R₃ die genannte Bedeutung hat, überführt, und den Ester in Gegenwart einer Base zum Endprodukt hydrolysiert.

Die Phosphonoessigsäuretrialkylester können durch Umsetzung von einem Trialkoxyphosphin mit einem Halogen-essigsäureester erhalten werden (Houben-Weyl, Methoden der organischen Chemie, 4.Auflage, Band: Phosphorverbindungen I, S.173-176).

Zweckmässig wird als Phosphonoessigsäuretrialkylester Phosphonoessigsäuretriethylester oder Diethylphosphonoessigsäureisopropylester verwendet. Vorzugsweise wird Phosphonoessigsäuretriethylester angewendet.

Zweckmässig erfolgt die Umsetzung des Phosphonoessigsäuretrialkylesters mit einer stöchiometrischen Menge Isobutylenoxid.

Die Umsetzung des Phosphonoessigsäuretrialkylesters mit Isobutylenoxid wird in Gegenwart einer Base durchgeführt. Als Base kann beispielsweise ein Alkalihydrid, wie z.B. Natriumhydrid, Kaliumhydrid oder Lithiumhydrid, angewendet werden.
Zweckmässig wird die Base aequimolar zu dem Phosphonoessigsäuretrialkylester eingesetzt.

Die Reaktionstemperatur liegt zweckmässig zwischen 100 und 140°C, vorzugsweise zwischen 110 und 130°C.

Als Lösungsmittel können hochsiedende aromatische Kohlenwasserstoffe oder polare Lösungsmittel verwendet werden. Beispielsweise können als hochsiedende aromatische Kohlenwasserstoffe Xylol oder Xylol-Isomerengemische angewendet werden.
Als polares Lösungsmittel können beispielsweise Glykolether, wie z.B. Diethylenglykoldimethylether, verwendet werden. Vorzugsweise wird als Lösungsmittel ein hochsiedender aromatischer Kohlenwasserstoff, wie z.B. o-Xylol, angewendet.

Der R,S-2,2-Dimethylcyclopropancarbonsäure-C₁-C₄-alkylester, wird dann zweckmässig ohne Isolation, in Gegenwart einer Base zum gewünschten R,S-2,2-DMCPCS hydrolysiert.

Zweckmässig verwendet man als Base für die Esterhydrolyse ein Alkalihydroxid.

Als Alkalihydroxid kann beispielsweise Natriumhydroxid oder Kaliumhydroxid angewendet werden.

Die Esterhydrolyse wird zweckmässig mit 1 bis 2 Mol Base, vorzugweise mit 1,2 bis 1,5 Mol, bezogen auf 1 Mol R,S-2,2-Dimethylcyclopropancarbonsäureester durchgeführt.

Zweckmässig erfolgt die Esterhydrolyse bei einer Temperatur von 40°C bis zur Rückflusstemperatur des eingesetzten Lösungsmittels, vorzugsweise bei Rückflusstempratur.

Die Esterhydrolyse wird zweckmässig in einem Gemisch von einem der genannten hochsiedenden aromatischen Kohlenwasserstoffe mit einer wässrigen alkoholischen Lösung durchgeführt. Als Alkohole können niedrige Alkohole, wie beispielsweise Methanol oder Ethanol,angewendet werden.

Die R,S-2,2-Dimethylcyclopropancarbonsäure kann nach 2 bis 3 Stunden Reaktionszeit, beispielsweise durch Ansäuern des Reaktionsgemisches und anschliessende Extraktion, isoliert werden.

### Beispiel

### Herstellung von R,S-2,2-Dimethylcyclopropancarbonsäure

In einem 150 ml Dreihalskolben wurden 4,8 g (0,16 mol) Natriumhydrid (80%ig in Weissöl) in 20,0 ml o-Xylol vorgelegt und innerhalb von 30 Minuten mit einer Lösung von 36,1 g (0,16 mol) Phosphonoessigsäuretriethylester in 35,0 ml o-Xylol tropfenweise bei Raumtemperatur versetzt. Sobald die H₂-Entwicklung beendet war, wurden 10,8 g (0,15 mol) Isobutylenoxid (BASF AG) in 20,0 ml o-Xylol zugegeben. Anschliessend wurde die Reaktionsmischung 2 Stunden auf 120°C erhitzt, auf Raumtemperatur abgekühlt und mit 50,0 ml Wasser versetzt. Die untere wässrige Phase wurde abgetrennt. Die Xylol-Phase wurde mit einer Lösung von 8,0 g NaOH in 30,0 ml Wasser und 50,0 ml Ethanol versetzt und 2 Stunden zum Rückfluss erhitzt. Anschliessend wurde die organische Phase abgetrennt. Die wässrige Phase wurde mit konz. HCl auf pH 1 eingestellt und zweimal mit je 50,0 ml n-Hexan extrahiert. Die Hexan-Phasen wurden vereinigt, getrocknet und eingedampft. Der Rückstand wurde im Wasserstrahlvakuum destilliert. Man erhielt 8,8 g farblose R,S-2,2-Dimethylcyclopropancarbonsäurelösung mit einem Gehalt von 98% R,S-2,2-Dimethylcyclopropancarbonsäure und einem Siedepunkt von 90°C (2,5 mbar), was einer Ausbeute von 50%, bezogen auf eingesetztes Isobutylenoxid, entsprach.

## Patentansprüche

1. Verfahren zur Herstellung von R,S-2,2-Dimethylcyclopropancarbonsäure der Formel 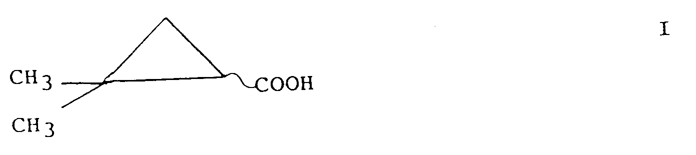 dadurch gekennzeichnet, dass man Isobutylenoxid der Formel 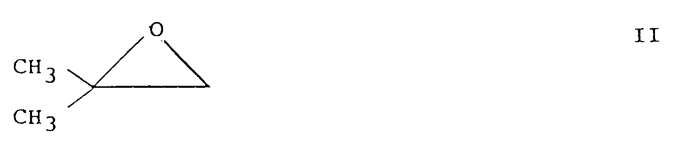 mit einem Phosphonoessigsäuretrialkylester der Formel 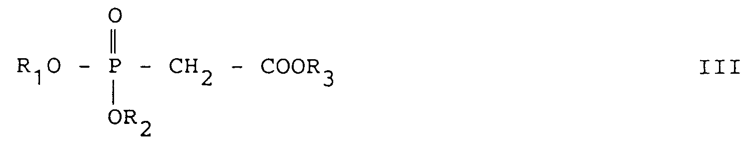 worin R₁, R₂ und R₃ gleich oder verschieden sind und eine C₁-C₄-Alkylgruppe, verzweigt oder unverzweigt, bedeuten, in Gegenwart einer Base in einen R,S-2,2-Dimethylcyclopropancarbonsäure-C₁-C₄-alkylester der allgemeinen Formel 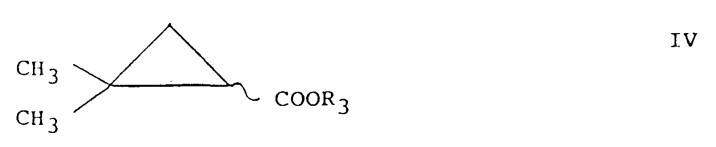 worin R₃ die genannte Bedeutung hat, überführt und den Ester in Gegenwart einer Base zum Endprodukt hydrolysiert.

2. Verfahren nach Patentanspruch 1, dadurch gekennzeichnet, dass man als Phosphonoessigsäuretrialkylester Phosphonoessigsäuretriethylester verwendet.

3. Verfahren nach den Patentansprüchen 1 und 2, dadurch gekennzeichnet, dass man den Phosphonoessigsäuretrialkylester in Gegenwart eines Alkalihydrids als Base bei einer Temperatur von 100 bis 140 °C umsetzt.

4. Verfahren nach Patentanspruch 1, dadurch gekennzeichnet, dass man die Esterhydrolyse in Gegenwart eines Alkalihydroxids als Base bei einer Temperatur von 40°C bis zur Rückflusstemperatur des Lösungsmittels durchgeführt.

5. Verfahren nach einem der Patentansprüche 1 bis 4, dadurch gekennzeichnet, dass man das Verfahren ohne Isolation der Zwischenstufe IV durchgeführt.

## Claims

1. A process for the preparation of R,S-2,2-dimethylcyclopropane carboxylic acid of the formula: 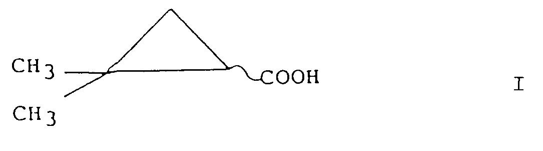 characterized in that isobutylene oxide of the formula: 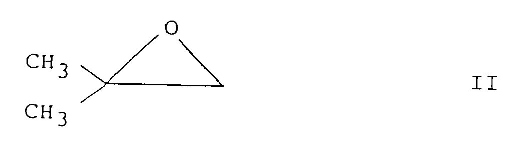 is converted with a phosphonoacetic acid trialkyl ester of the formula: 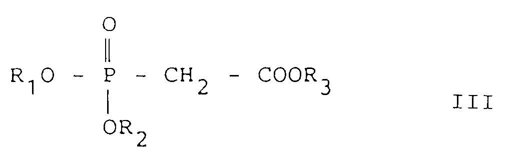 wherein R₁, R₂ and R₃ have the same or different meanings, each representing a branched or unbranched C₁-C₄ alkyl group; in the presence of a base into an R,S-2,2-dimethylcyclopropane carboxylic acid C₁-C₄ alkyl ester of the general formula: 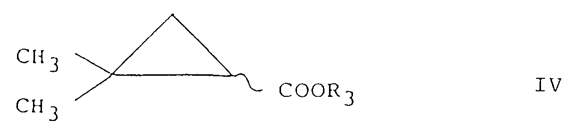 wherein R₃ has the above meaning; and hydrolizing said ester in the presence of a base to the final product.

2. The process according to Claim 1, characterized in that phosphonoacetic acid triethyl ester is used as a phosphonoacetic acid trialkyl ester.

3. The process according to Claims 1 and 2, characterized in that the phosphonoacetic acid trialkyl ester is reacted in the presence of an alkali hydride as a base at a temperature of from 100 to 140°C.

4. The process according to Claim 1, characterized in that ester hydrolysis is conducted in the presence of an alkali hydroxide as a base at a temperature of from 40°C to reflux temperature of the solvent.

5. The process according to any one of Claims 1 to 4, characterized in that the process is conducted without isolation of intermediate product IV.

## Revendications

1. Procédé pour la préparation de l'acide R,S-2,2-diméthylcyclopropanecarboxylique de la formule 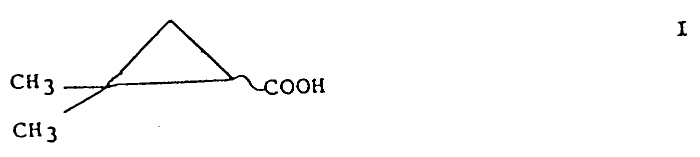 caractérisé en ce que l'on fait passer l'oxyde d'isobutylène de la formule 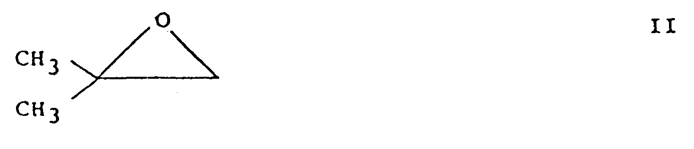 avec un trialkylester de l'acide phosphonoacétique de la formule 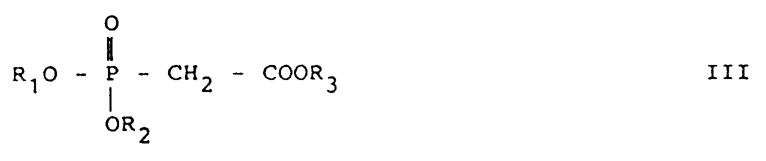 dans laquelle R₁, R₂ et R₃ sont identiques ou différents et signifient un groupe alkyle en C₁-C₄, ramifié ou non ramifié, en présence d'une base? dans un alkylester en C₁-C₄ de l'acide R,S-2,2-diméthylcyclopropanecarboxylique de la formule générale 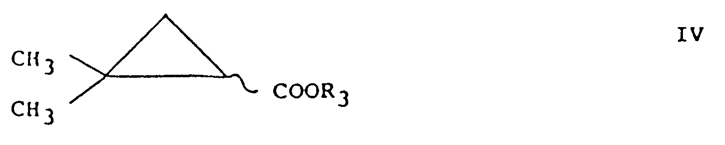 dans laquelle R₃ a la signification indiquée et en ce que l'on hydrolyse l'ester en présence d'une base pour donner le produit final.

2. Procédé selon la revendication 1, caractérisé en ce qu'en tant que trialkylester de l'acide phosphonoacétique, on utilise le triéthylester de l'acide phosphonoacétique.

3. Procédé selon les revendications 1 et 2, caractérisé en ce que l'on met en réaction le trialkylester de l'acide phosphonoacétique en présence d'un hydride alcalin comme base à une température de 100 à 140°C.

4. Procédé selon la revendication 1, caractérisé en ce que l'on conduit l'hydrolyse de l'ester en présence d'un hydroxyde alcalin comme base à une température de 40°C jusqu'à la température de reflux du solvant.

5. Procédé selon l'une des revendications 1 à 4, caractérisé en ce que l'on conduit le procédé sans isolation de l'étape intermédiaire IV.
